# EUROPEAN PATENT APPLICATION

(11) **EP 3 977 971 A1**
(43) Date of publication of application: **06.04.2022**
(21) Application number: 20199597.4
(22) Date of filing: 01.10.2020
(51) Int. Cl.: A61F 15/00

(54) **WRAP BANDAGE STORAGE CONTAINER**

(71) Applicant: Webster, Brian, Donald, Edmonton, Alberta T5X 6H8 (CA)
(72) Inventor: Webster, Brian, Donald, Edmonton, Alberta T5X 6H8 (CA)
(74) Representative: Gulde & Partner

(57) **Abstract**

The present disclosure refers to a storage container for a linear flat material wherein the storage container provides the winding and dispensing of the linear flat material. The storage container comprises a body member, said body member being manufactured from a lightweight rigid material, said body member having a plurality of walls forming an interior volume and an entry slot, said entry slot being formed in one of said plurality of walls of said body member, said entry slot extending substantially across said one of said plurality of walls, said entry slot configured to receive the linear flat material therethrough. The storage container further comprises a shaft member, said shaft member being disposed in said interior volume of said body member, said shaft member being rotatably mounted to one of said plurality of walls of said body member wherein said shaft member is rotated by a user of the storage container so as to facilitate the winding or dispensing of flat linear material therefrom.

## Description

### FIELD OF THE INVENTION

The present invention relates generally to health and wellness, more specifically but not by way of limitation, a storage container for elastic bandage wherein the storage container is configured to provide winding and storage of elastic bandage so as to more efficiently store and subsequently dispense linear elastic bandages and similar material.

### BACKGROUND

As is known in the art, wrap bandages are linear in configuration and are utilized to treat many different symptoms and/or ailments. Conventional wrap bandages are manufactured from a stretchable material and are used to create localized pressure on an area of the body such as but not limited to a knee or elbow. These bandages are commonly used to treat muscle sprains by reducing the flow of blood through the application of pressure which can reduce the swelling of the area. For some treatment protocols, elastic bandage are utilized in conjunction with splints. This is a common technique for arm fractures and the like where swelling may occur and use of this technique typically precedes application of a fiberglass cast or other device.

One issue with the management and maintenance of the elastic bandage occurs subsequent being removed from its original packing. For packaging, the elastic bandage is typically mechanically rolled into a tight roll and seal in a plastic package for retail. Ensuing removal from the packaging the compression of the roll will relax. Further, once the elastic bandage roll has been deployed and utilized on a patient the elastic wraps are quite cumbersome to manage. Attempts are made to manually roll but this results twisting of the bandage which must be straightened out in order to apply properly to another individual. Manually rolling the elastic bandages typically yields a disorganized pile, which makes storage more challenging and further makes efficient subsequent use difficult.

Accordingly, there is a need for a wrap bandage storage container that provides efficient winding and dispensing of a wrap bandage wherein the wrap bandage storage container further inhibits twisting of the bandage during retrieval and dispensing.

### SUMMARY OF THE INVENTION

It is the object of the present invention to provide a wrap bandage storage container configured to receive and store linear elastic wrap bandages that further includes a body member having a first wall and a second wall integrally formed to create an interior volume.

Another object of the present invention is to provide a wrap bandage storage container configured to provide storage of a wrap bandage or similar material that further includes a shaft member disposed in the interior volume.

A further object of the present invention is to provide a wrap bandage storage container configured to receive and store linear elastic wrap bandages wherein the shaft member is rotatably mounted on the first wall of the body member and perpendicular therewith.

Still another object of the present invention is to provide a wrap bandage storage container configured to provide storage of a wrap bandage or similar material wherein the shaft member further includes a receiving slot configured to have a portion of wrap bandage journaled therethrough.

An additional object of the present invention is to provide a wrap bandage storage container configured to receive and store linear elastic wrap bandages wherein the body member is formed to have a closed side and an open side.

Yet a further object of the present invention is to provide a wrap bandage storage container configured to provide storage of a wrap bandage or similar material wherein the second wall includes a first end portion and a second end portion.

Another object of the present invention is to a provide a wrap bandage storage container configured to receive and store linear elastic wrap bandages wherein the first end portion and second end portion of the second wall are formed to create an entry slot.

An alternate object of the present invention is to provide a wrap bandage storage container configured to provide storage of a wrap bandage or similar material wherein the entry slot is formed to receive a wrap bandage therethrough.

Still a further object of the present invention is to provide a wrap bandage storage container configured to receive and store linear elastic wrap bandages wherein the entry slot includes arcuate portions that are operable to inhibit twisting of the wrap bandage during the retrieval and storage process.

An additional object of the present invention is to provide a wrap bandage storage container configured to provide storage of a wrap bandage or similar material wherein the shaft member is operably coupled to a handle member that is opposedly located on the first wall of the body member.

According to a first embodiment, there is disclosed a storage container for a linear flat material wherein the storage container provides the winding and dispensing of the linear flat material. The storage container comprises a body member, said body member being manufactured from a lightweight rigid material, said body member having a plurality of walls forming an interior volume and an entry slot, said entry slot being formed in one of said plurality of walls of said body member, said entry slot extending substantially across said one of said plurality of walls, said entry slot configured to receive the linear flat material therethrough. The storage container further comprises a shaft member, said shaft member being disposed in said interior volume of said body member, said shaft member being rotatably mounted to one of said plurality of walls of said body member wherein said shaft member is rotated by a user of the storage container so as to facilitate the winding or dispensing of flat linear material therefrom.

The shaft member may further include a receiving slot, said receiving slot extending substantially along said shaft member. The body member may further include a front region, said front region being integrally formed in said plurality of walls of said body member, said front region providing a structure to improve introduction of the linear flat material into the interior volume of the body member. The storage container may further include a handle member, said handle member being operably coupled to said shaft member, said handle member being located exteriorly to the body member, said handle member operable to provide rotation of said shaft member. The plurality of walls of said body member may further include finger indentations formed therein, said finger indentation being proximate said front region of said body member.

According to a further embodiment, there is disclosed a storage container that is configured to provide winding, storage and dispensing of wrap bandage comprising a body member, said body member having a first wall and a second wall, said first wall and said second wall of said body member configured to form an interior volume of said body member, said body member having a first side and a second side, said body member being manufactured from a lightweight rigid material, said second wall being perpendicular to said first wall and extending outward therefrom. The storage container further includes a shaft member, said shaft member being disposed within said interior volume of said body, said shaft member being mounted to said first wall and being perpendicular therewith, said shaft member being rotatable so as to facilitate the winding and dispensing of the wrap bandage, said shaft member further having a slot configured to receive a portion of the wrap bandage therethrough wherein said second wall further includes a first end and a second end, said first end and said second end of said second wall having an entry slot intermediate thereto, said entry slot being configured to have the wrap bandage journaled therethrough.

The body member may further include a front region, said front region being formed in said second wall, said front region configured to protrude outward from said body member. The second side of said body member may include an opening, said opening providing access to the interior volume of said body member. The first end and said second end of said second wall may be formed so as to be arcuately opposed in shape. The second wall further may include a first finger indentation and a second finger indentation, said first finger indentation being proximate said first end of said second wall, said second finger indentation being proximate said second end of said second wall. The storage container may further include a handle member, said handle member being operably coupled to said shaft member, said handle member being located exteriorly to the body member, said handle member operable to provide rotation of said shaft member. The second wall may be contiguously formed with said first wall proximate a perimeter edge of said first wall. The body member may be manufactured to have a width of approximately two inches (5.08 cm).

According to a first embodiment, there is disclosed a wrap bandage storage container that is configured to provide winding, storage and dispensing of wrap bandage comprising a body member, said body member having a first wall and a second wall, said second wall being contiguous with a perimeter edge of said first wall and perpendicular thereto, said second wall further includes a first end and a second end, said first wall and said second wall of said body member configured to form an interior volume of said body member, said body member having a first side and a second side, said body member being manufactured from a lightweight rigid material. The wrap bandage storage container further includes an entry slot, said entry slot being intermediate said first end and said second end of said second wall, said entry slot extending substantially across said second wall, said entry slot configured to have the wrap bandage pass therethrough and a shaft member, said shaft member being disposed within said interior volume of said body, said shaft member being mounted to said first wall and being perpendicular therewith, said shaft member being rotatable so as to facilitate the winding and dispensing of the wrap bandage, said shaft member further having a slot configured to receive a portion of the wrap bandage therethrough wherein said body member further includes a front region, said front region being formed in said second wall, said front region configured to protrude outward from said body member so as to improve introduction of the wrap bandage into the interior volume of said body member.

The wrap bandage storage container may further include a handle member, said handle member being operably coupled to said shaft member, said handle member being located exteriorly to the body member, said handle member having a first portion and a second portion being perpendicular to each other, said handle member operable to provide rotation of said shaft member. The second wall may further include a first finger indentation and a second finger indentation, said first finger indentation being proximate said first end of said second wall, said second finger indentation being proximate said second end of said second wall, said first finger indentation and said second finger indentation being located opposedly with respect to said entry slot. The second side of said body member may further include an opening, said opening of said second side providing access to the interior volume of the body member. The body member may be manufactured to have a width of approximately two inches (5.08 cm).

To the accomplishment of the above and related objects the present invention may be embodied in the form illustrated in the accompanying drawings. Attention is called to the fact that the drawings are illustrative only. Variations are contemplated as being a part of the present invention, limited only by the scope of the claims.

### BRIEF DESCRIPTION OF THE DRAWINGS

A more complete understanding of the present invention may be had by reference to the following Detailed Description and appended claims when taken in conjunction with the accompanying Drawings wherein:

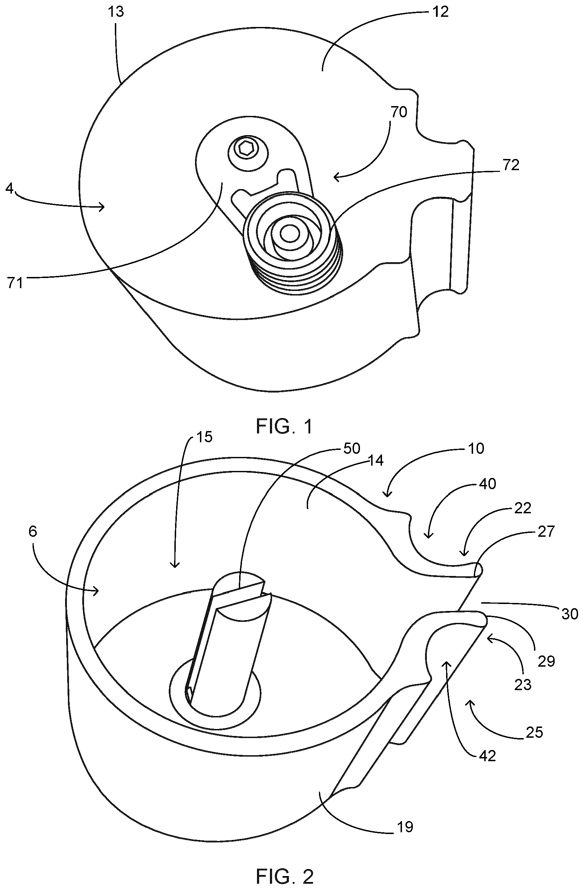

Figure 1 is a side view of a preferred embodiment of the present invention; and
Figure 2 is a side view of the present invention showing the interior volume thereof; and

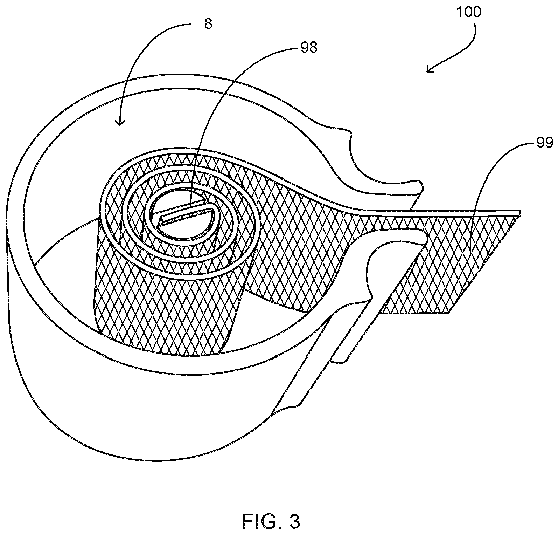

Figure 3 is a side view of the present invention showing exemplary wrap bandage engaged therewith.

### DETAILED DESCRIPTION

Referring now to the drawings submitted herewith, wherein various elements depicted therein are not necessarily drawn to scale and wherein through the views and figures like elements are referenced with identical reference numerals, there is illustrated a wrap bandage storage container 100 constructed according to the principles of the present invention.

An embodiment of the present invention is discussed herein with reference to the figures submitted herewith. Those skilled in the art will understand that the detailed description herein with respect to these figures is for explanatory purposes and that it is contemplated within the scope of the present invention that alternative embodiments are plausible. By way of example but not by way of limitation, those having skill in the art in light of the present teachings of the present invention will recognize a plurality of alternate and suitable approaches dependent upon the needs of the particular application to implement the functionality of any given detail described herein, beyond that of the particular implementation choices in the embodiment described herein. Various modifications and embodiments are within the scope of the present invention.

It is to be further understood that the present invention is not limited to the particular methodology, materials, uses and applications described herein, as these may vary. Furthermore, it is also to be understood that the terminology used herein is used for the purpose of describing particular embodiments only, and is not intended to limit the scope of the present invention. It must be noted that as used herein and in the claims, the singular forms "a", "an" and "the" include the plural reference unless the context clearly dictates otherwise. Thus, for example, a reference to "an element" is a reference to one or more elements and includes equivalents thereof known to those skilled in the art. All conjunctions used are to be understood in the most inclusive sense possible. Thus, the word "or" should be understood as having the definition of a logical "or" rather than that of a logical "exclusive or" unless the context clearly necessitates otherwise. Structures described herein are to be understood also to refer to functional equivalents of such structures. Language that may be construed to express approximation should be so understood unless the context clearly dictates otherwise.

References to "one embodiment", "an embodiment", "exemplary embodiments", and the like may indicate that the embodiment(s) of the invention so described may include a particular feature, structure or characteristic, but not every embodiment necessarily includes the particular feature, structure or characteristic.

Now referring in particular to the Figures submitted herewith, the wrap bandage storage container 100 includes a body member 10 wherein the body member 10 is integrally formed utilizing a first wall 12 and a second wall 14 through suitable techniques. The body member 10 is manufactured from a rigid lightweight material such as but not limited to plastic or metal. While no particular size of the body member 10 is required, it is contemplated within the scope of the present invention that the body member 10 is approximately two inches (5.08 cm) in width so as to easily fit into the palm of a human hand. The first wall 12 and second wall 14 are configured to create interior volume 15 wherein the interior volume 15 is of suitable size to accommodate exemplary wrap bandage 99 therein. It should be understood within the scope of the present invention that while elastic bandages are the preferred material to be stored and dispensed using the wrap bandage storage container 100 that any other material similar in structure could be stored using the wrap bandage storage container 100.

The first wall 12 includes perimeter edge 13 defining the shape thereof. The second wall 14 is contiguous with the perimeter edge 13 and extends outward perpendicular therefrom so as to create the interior volume 15. The wrap bandage storage container 100 includes a first side 4 and a second side 6 wherein the first side 4 has the first wall 12 and the second side 6 has opening 8 that provides access to the interior volume 15. The opening 8 allows a user to insert their hands partially into the interior volume 15 so as to engage the shaft member 50 and exemplary wrap bandage 99. The aforementioned is executed during initial loading of the exemplary wrap bandage. While the body member 10 is illustrated herein as having an opening 8 on the second side 6, it is contemplated within the scope of the present invention that the second side 6 could be closed off utilizing a wall similar to that of the first wall 12.

The second wall 14 is mateably shaped with the perimeter edge 13 of the first wall 12 and is substantially annular in form. The second wall 14 includes a first end 22 and a second end 23 located in the front region 25 of the body member 10. The first end 22 and second end 23 terminate so as to form an entry slot 30 therebetween. The entry slot 30 extends substantially across the second wall 14 and is configured to receive the exemplary wrap bandage 99 therethrough. The first end 22 and second end 23 are formed so as to arcuately oppose so as to provide a smooth entry for the exemplary wrap bandage 99. It should be understood that the arcuate opposition of the first end 22 and second end 23 are the portions 27,29 that curve away from each other. It is further contemplated within the scope of the present invention that the entry slot 30 is formed to have a tolerance that assists in the untwisting and/or inhibits twisting of the exemplary wrap bandage 99 as during the winding process. The second wall 14 has formed on the outer surface 19 thereof opposing finger indentations 40,42. The finger indentations 40,42 provide an area of engagement for at least two fingers of a human hand during the holding of the wrap bandage storage container 100 in the winding/dispensing process. It should be understood within the scope of the present invention that the second wall 14 could be formed without the finger indentations 40,42 in the front region 25. The front region 25 protrudes outward from the body member 10 so as to provide a structure of improved introduction of the exemplary wrap bandage 99 into the wrap bandage storage container 100.

The shaft member 50 is rotatably mounted within the interior volume 15. The shaft member 50 is secured to the first wall 12 and is manufactured from a rigid lightweight material such as but not limited to plastic or metal. The shaft member 50 includes slot 55 formed therein. Slot 55 is configured to receive portion 98 of the exemplary wrap bandage 99 at the initial point of the winding process. The slot 55 extends substantially the length of the shaft member 50. The shaft member 50 is operably coupled to handle member 70. Handle member 70 is located on the opposing side of the first wall 12 from the shaft member 50. Handle member 70 includes a first portion 71 and second portion 72 that are operably coupled. The first portion 71 and second portion 72 are formed in a manner so as to facilitate engagement of the second portion 72 with a hand of a user in order to provide rotation of the handle member 70. Rotation of the handle member 70 can occur in both a clockwise and a counterclockwise direction depending upon whether a user of the wrap bandage storage container 100 is winding or dispensing the exemplary wrap bandage 99.

While a handle member 70 is illustrated and discussed herein for providing operation of the shaft member 70, it is further contemplated within the scope of the present invention that alternate elements could be employed to provide rotation of the shaft member 50. By way of example but not limitation, a small battery operated electric motor could be provided to perform the desired function. Furthermore, while the embodiment illustrated herein is configured to be grasped and held by a user's hands, it is contemplated within the scope of the present invention that the body member 10 could include a wall mount member so as to mount the wrap bandage storage container 100 to a wall. Lastly, it is additionally contemplated within the scope of the present invention that the body member 10 could be formed in alternate shapes and/or sizes.

In the preceding detailed description, reference has been made to the accompanying drawings that form a part hereof, and in which are shown by way of illustration specific embodiments in which the invention may be practiced. These embodiments, and certain variants thereof, have been described in sufficient detail to enable those skilled in the art to practice the invention. It is to be understood that other suitable embodiments may be utilized and that logical changes may be made without departing from the spirit or scope of the invention. The description may omit certain information known to those skilled in the art. The preceding description is, therefore, not intended to be limited to the specific forms set forth herein, but on the contrary, it is intended to cover such alternatives, modifications, and equivalents, as can be reasonably included within the spirit and scope of the invention.

## Claims

1. A storage container (100) for a linear flat material wherein the storage container provides the winding and dispensing of the linear flat material wherein the storage container (100) comprises:
a body member (10), said body member (10) being manufactured from a lightweight rigid material, said body member (10) having a plurality of walls forming an interior volume (15);
an entry slot (30), said entry slot (30) being formed in one of said plurality of walls of said body member (10), said entry slot (30) extending substantially across said one of said plurality of walls, said entry slot (30) configured to receive the linear flat material therethrough;
a shaft member (50), said shaft member (50) being disposed in said interior volume (15) of said body member (10), said shaft member (50) being rotatably mounted to one of said plurality of walls of said body member (10); and
wherein said shaft member (50) is rotated by a user of the storage container (100) so as to facilitate the winding or dispensing of flat linear material therefrom.

2. The storage container (100) as recited in claim 1, wherein said shaft member (50) further includes a receiving slot, said receiving slot extending substantially along said shaft member (50).

3. The storage container (100) as recited in claim 3, wherein said body member (10) further includes a front region (25), said front region (25) being integrally formed in said plurality of walls of said body member (10), said front region (25) providing a structure to improve introduction of the linear flat material into the interior volume (15) of the body member (10).

4. The storage container (100) as recited in claim 3, wherein said front region (25) is configured to protrude outward from said body member (10).

5. The storage container (100) as recited in claim 3, and further including a handle member (70), said handle member (70) being operably coupled to said shaft member (50), said handle member (70) being located exteriorly to the body member (10), said handle member (70) operable to provide rotation of said shaft member (50).

6. The storage container (100) as recited in claim 5, wherein said plurality of walls of said body member (10) further include finger indentations (40, 42) formed therein, said finger indentations (40, 42) being proximate said front region (25) of said body member (10).

7. The storage container (100) as recited in claim 1, wherein said body member (10) has a first wall (12) and a second wall (14), said first wall (12) and said second wall (14) of said body member (10) configured to form an interior volume (15) of said body member (10), said body member (10) having a first side (4) and a second side (6) said second wall (14) being perpendicular to said first wall (12) and extending outward therefrom, wherein said shaft member (50) is mounted to said first wall (12) and being perpendicular therewith and wherein said second wall (14) further includes a first end (22) and a second end (23), said first end (22) and said second end (23) of said second wall (14) having an entry slot (30) intermediate thereto.

8. The storage container (100) as recited in claim 7, wherein said second side (6) of said body member (10) includes an opening (8), said opening (8) providing access to the interior volume (15) of said body member (10).

9. The storage container (100) as recited in claim 8, wherein said first end (22) and said second end (23) of said second wall (14) are formed so as to be arcuately opposed in shape.

10. The storage container (100) as recited in claim 7, wherein said second wall (14) is contiguously formed with said first wall (12) proximate a perimeter edge (13) of said first wall (12).

11. The storage container (100) as recited in claim 10, wherein said body member (10) is manufactured to have a width of approximately 5.08 cm.
